(19) Europäisches Patentamt
European Patent Office
Office européen des brevets

(11) Veröffentlichungsnummer: **0 189 043**
**B1**

(12) # EUROPÄISCHE PATENTSCHRIFT

(45) Veröffentlichungstag der Patentschrift: **08.03.89**

(51) Int. Cl.⁴: **C 07 D 271/08,** C 07 D 413/04, A 01 N 47/36

(21) Anmeldenummer: **86100094.1**

(22) Anmeldetag: **07.01.86**

(54) **Substituierte Furazane.**

(30) Priorität: **19.01.85 DE 3501723**

(43) Veröffentlichungstag der Anmeldung: **30.07.86 Patentblatt 86/31**

(45) Bekanntmachung des Hinweises auf die Patenterteilung: **08.03.89 Patentblatt 89/10**

(84) Benannte Vertragsstaaten: **AT BE CH DE FR GB IT LI NL**

(56) Entgegenhaltungen:
**EP-A-0 132 680**
**DE-A-2 436 179**
**US-A-4 449 998**

**CHEMICAL ABSTRACTS, Band 79, Nr. 17, 29. Oktober 1973, Seite 420, Abstract Nr. 105152k, Columbus, Ohio, US; G. WESTPHAL et al. "3-Amino-1,2,5-oxadiazoles"**
**CHEMICAL ABSTRACTS, Band 94, Nr. 5, 2. Februar 1981, Seite 119, Abstract Nr. 25636r, Columbus, Ohio, US; R. CALVINO et al. "Antimicrobial properties of some furazan and furoxan derivatives"**

(73) Patentinhaber: **BAYER AG, Konzernverwaltung RP Patentabteilung, D-5090 Leverkusen 1 Bayerwerk (DE)**

(72) Erfinder: **Sirrenberg, Wilhelm, Dr., Wuppertaler Strasse 21, D-4322 Sprockhövel (DE)**
Erfinder: **Braden, Rudolph, Dr., Nothauserfeld 1, D-5068 Odenthal (DE)**
Erfinder: **Ziemann, Heinz, Dr., Am Wiesenberg 10, D-5653 Leichlingen (DE)**
Erfinder: **Zoebelein, Gerhard, Dr., Domblick 46, D-5090 Leverkusen 31 (DE)**
Erfinder: **Pflüger, Wolfgang, Dr., Am Treppchen 7, D-5653 Leichlingen (DE)**

**EP 0 189 043 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft neue substituierte Furazane, Verfahren zu ihrer Herstellung und ihre Verwendung als Schädlingsbekämpfungsmittel, insbesondere als Insektizide und Akarizide.

Es ist bisher noch nicht bekannt, daß substituierte Furazane als Schädlingsbekämpfungsmittel verwendet werden können, bisher ist lediglich bekannt, daß bestimmte Heterocyclen wie z. B. 3-Phenyl-2-phenylimino-4,5-bis-(trifluormethylimino-thiazolidin, insektizide Eigenschaften besitzen (vergl. z. B. DE-AS-2 062 348). Weiterhin wurden bestimmte Furazane, Furoxane und Isoxazole ohne Wirkungsangabe oder unter der Angabe von mikrobiziden oder herbiziden Wirkungen beschrieben (vgl. CA 79, 1973, 105152k, CA 94, 1981, 25636r und DE-A-2 436 179).

Es wurden die neuen substituierten Furazane der Formel (I)

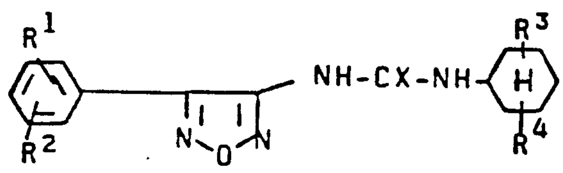

(I)

in welcher

$R^1$ und $R^2$ gleich oder verschieden sind und für Wasserstoff, Halogen, $C_1$-$C_6$-Alkyl, $C_1$-$C_6$-Alkoxy, $C_1$-$C_6$-Alkylthio, Halogen-$C_1$-$C_6$-Alkyl, Halogen-$C_1$-$C_6$-Alkoxy, Halogen-$C_1$-$C_6$-Alkylthio oder für gegebenenfalls durch Halogen, Nitro, Cyano, $C_1$-$C_4$-Alkyl, $C_1$-$C_4$-Alkoxy, $C_1$-$C_4$-Alkylthio, Halogen-$C_1$-$C_4$-Alkyl, Halogen-$C_1$-$C_4$-Alkoxy und/oder Halogen-$C_1$-$C_4$-Alkylthio substituiertes Aryloxy mit 6 bis 10 Kohlenstoffatomen im Arylteil stehen, oder

$R^1$ und $R^2$ gemeinsam für einen gegebenenfalls durch Halogen und/oder $C_1$-$C_2$-Alkyl substituierten $C_1$-$C_3$-Alkylen-Rest, welcher durch 1 oder 2 Sauerstoffatome unterbrochen wird, stehen,

$R^3$ und $R^4$ gleich oder verschieden sind und für Wasserstoff, $C_1$-$C_6$-Alkyl, $C_1$-$C_6$-Alkoxy, Halogen-$C_1$-$C_6$-Alkyl oder Halogen-$C_1$-$C_4$-Alkoxy stehen und

X für Sauerstoff oder Schwefel steht, gefunden.

Weiterhin wurde gefunden, daß die neuen substituierten Furazane der Formel (I) erhalten werden, indem man

a) 3-Amino-1,2,5-oxadiazole der Formel (II)

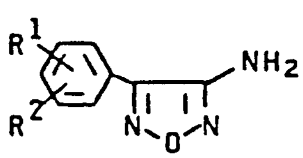

(II)

in welcher
$R^1$ und $R^2$ die oben angegebenen Bedeutungen haben,
mit Iso(thio)cyanaten der Formel (III)

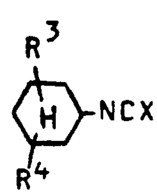

(III)

in welcher
X, $R^3$ und $R^4$ die oben angegebenen Bedeutungen haben,
gegebenenfalls in Gegenwart von Katalysatoren und gegebenenfalls in Gegewart von Verdünnungsmitteln umsetzt, oder

b) 3-Iso(thio)cyanato-1,2,5-oxadiazole der Formel (IV),

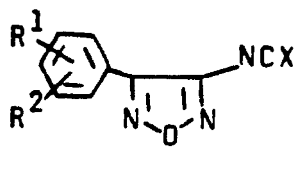

(IV)

in welcher
X, $R^1$ und $R^2$ die oben angegebenen Bedeutungen haben,
mit Aminen der Formel (V)

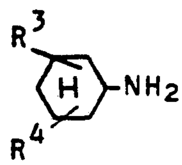

(V)

in welcher
$R^3$ und $R^4$ die oben angegebenen Bedeutungen haben,
gegebenenfalls in Gegenwart von Katalysatoren und gegebenenfalls in Gegenwart von Verdünnungsmitteln umsetzt.

Die Verbindungen der Formel (I) können in verschiedenen geometrischen und optischen Isomerenformen vorliegen, je nach Anordnung der Substituenten die an den Cyclohexyl-Rest gebunden sind; vorzugsweise fallen sie in einem wechselnden Isomerenverhältnis an. Die vorliegende Erfindung betrifft sowohl die einzelnen Isomeren als auch die Isomerengemische.

Alkyl $R^1$, $R^2$, $R^3$ und $R^4$ enthalten im Alkylteil geradkettiges oder verzweigtes Alkyl mit 1 bis 6, insbesondere 1 bis 4 Kohlenstoffatomen. Beispielhaft seien Methyl, Ethyl, n-Propyl, i-Propyl, n-Butyl, i-Butyl, sec.-Butyl und tert.-Butyl genannt.

Alkoxy $R^1$, $R^2$, $R^3$ und $R^4$ enthalten im Alkylteil geradkettiges oder verzweigtes Alkyl mit 1 bis 6, insbesondere 1 bis 4 Kohlenstoffatomen. Beispielhaft seien Methoxy, Ethoxy, n-Propoxy, i-Propoxy, n-Butoxy, i-Butoxy, sec.-Butoxy und tert.-Butoxy genannt.

Alkylthio $R^1$ und $R^2$ enthalten im Alkylteil geradkettiges oder verzweigtes Alkyl mit 1 bis 6, insbesondere 1 bis 4 Kohlenstoffatomen. Beispielhaft seien Methyl-, Ethyl-, n-Propyl, i-Propyl-, n-Butyl-, i-Butyl-, sec.-Butyl- und tert.-Butylthio genannt.

Halogenalkyl und Halogenalkoxy $R^1$, $R^2$, $R^3$ und $R^4$ und Halogenalkylthio $R^1$ und $R^2$ enthalten im Alkylteil geradkettiges oder verzweigtes Alkyl mit 1 bis 6, vorzugsweise 1 bis 4, insbesondere 1 bis 2 Kohlenstoffatomen und vorzugsweise 1 bis 6, insbesondere 1 bis 4 Halogenatome, wobei die Halogenatome gleich oder verschieden sind und als Halogenatome vorzugsweise Fluor, Chlor oder Brom, insbesondere Fluor, stehen.

Beispielhaft seien genannt Trifluormethyl, Chlordifluormethyl, Difluormethyl, Trifluormethylthio, Chlordifluormethylthio, Trifluormethylthio, Chlortrifluorethylthio, Tetrafluorethylthio, Trifluormethoxy, Difluormethoxy, Tetrafluorethoxy, Chlortrifluorethoxy und Bromtrifluorethoxy.

Als Aryloxy $R^1$ und $R^2$ steht Aryloxy mit 6 bis 10 Kohlenstoffatomen im Arylteil, vorzugsweise Phenoxy oder Naphthyloxy, insbesondere Phenoxy.

Die Aryloxyreste $R^1$ und $R^2$ können einen oder mehrere, vorzugsweise 1 bis 3, insbesondere 1 oder 2 gleiche oder verschiedene Substituenten tragen.

Als Substituenten seien aufgeführt:

Alkyl mit 1 bis 4, insbesondere 1 oder 2 Kohlenstoffatomen, wie Methyl, Ethyl, n- und i-Propyl und n-, i-, sec - und t-Butyl; Alkoxy mit 1 bis 4, insbesondere 1 oder 2 Kohlenstoffatomen, wie Methoxy, Ethoxy, n- und i-Propyloxy und n-, i-, sec.- und t-Butyloxy; Alkylthio mit 1 bis 4, insbesondere 1 oder 2 Kohlenstoffatomen, wie Methylthio, Ethylthio, n- und i-Propylthio und n-, i-, sec.- und t-Butylthio; Halogenalkyl mit 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor, stehen, wie Trifluormethyl; Halogenalkoxy mit 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor oder Brom, insbesondere Fluor, stehen, wie Trifluormethoxy; Halogen, vorzugswaise Fluor, Chlor, Brom oder Iod, insbesondere Chlor und Brom; Cyano und Nitro.

Alkylen in der Definition von $R^1$ und $R^2$, welches durch 1 oder 2 Sauerstoffatome unterbrochen wird, enthält 1 bis 3, insbesondere 1 oder 2 Kohlenstoffatome und kann durch $C_1$-$C_2$-Alkyl und/oder Halogen substituiert sein.

Halogen steht, wo nicht anders erläutert, für Fluor, Chlor, Brom und Iod, vorzugsweise für Chlor und Fluor, wobei die Halogenatome gleich oder verschieden sein können.

Die Reste $R^1$ und $R^2$ stehen vorzugsweise in der 2-, 4-, 2,4-, 3,4- oder 2,6-Position des Phenylringes.

Die Reste $R^3$ und $R^4$ stehen bevorzugt in der 3-, 4-, 3,4- oder 3,5-Position des Cyclohexylringes.

Die neuen Verbindungen der Formel (I) verfügen über Eigenschaften, die ihre Verwendung als Schädlingsbekämpfungsmittel ermöglichen, insbesondere zeichnen sie sich durch eine hervorragende insektizide und akarizide Wirksamkeit aus.

Bevorzugt werden die neuen substituierten Furazane der Formel (I), in welcher

| | |
|---|---|
| $R^1$ und $R^2$ | gleich oder verschieden sind und für Wasserstoff, Fluor, Chlor, Brom, $C_1$-$C_4$-Alkyl, $C_1$-$C_4$-Alkoxy, $C_1$-$C_4$-Alkylthio, Halogen-$C_1$-$C_4$-Alkyl, Halogen-$C_1$-$C_4$-Alkoxy, Halogen-$C_1$-$C_4$-Alkylthio oder für gegebenenfalls durch Halogen, Nitro, Cyano, $C_1$-$C_4$-Alkyl, $C_1$-$C_4$-Alkoxy, $C_1$-$C_4$-Alkylthio, Halogen-$C_1$-$C_4$-Alkyl, Halogen-$C_1$-$C_4$-Alkoxy und/oder Halogen-$C_1$-$C_4$-Alkylthio substituiertes Phenoxy stehen, oder |
| $R^1$ und $R^2$ | gemeinsam für einen gegebenenfalls durch Fluor, Chlor und/oder Methyl substituierten $C_1$-$C_3$-Alkylen-Rest, welcher durch 1 oder 2 Sauerstoffatome unterbrochen wird, stehen, |
| $R_3$ und $R_4$ | gleich oder verschieden sind und für Wasserstoff, $C_1$-$C_4$-Alkyl, $C_1$-$C_4$-Alkoxy, Halogen-$C_1$-$C_4$-Alkyl oder Halogen-$C_1$-$C_4$-Alkoxy stehen und |
| X | für Sauerstoff oder Schwefel steht. |

Ganz besonders bevorzugt werden die neuen substituierten Furazane der Formel (I), in welcher

| | |
|---|---|
| $R^1$ und $R^2$ | gleich oder verschieden sind und für Wasserstoff, Fluor, Chlor, Brom, Methoxy, Ethoxy, Methyl, Ethyl, n-Propyl, i-Propyl, n-Butyl, i-Butyl, sec.-Butyl, tert.-Butyl, Methylthio, Ethylthio, Trifluormethyl, Trifluormethoxy, Difluormethyl, Difluorethyl, Pentafluorethyl, Difluormethoxy, Chlordifluormethoxy, Chlortrifluorethoxy, Tetrafluorethoxy, Dichlordifluorethoxy, Difluortrichlorethoxy, Hexafluorpropoxy, Trifluormethylthio, Difluormethylthio, Chlordifluormethylthio, Chlortrifluorethylthio, Hexafluorpropylthio oder für gegebenenfalls durch Fluor, Chlor, Brom, Nitro, Cyano, Methyl, Ethyl, n-Propyl, i-Propyl, n-Butyl, i-Butyl, sec.-Butyl, tert.-Butyl, Methoxy, Ethoxy, n-Propoxy, i-Propoxy, n-Butoxy, i-Butoxy, sec.-Butoxy, tert.-Butoxy, Methylthio, Ethylthio, Trifluormethyl, Trifluormethoxy, Difluormethyl, Difluorethyl, Pentafluorethyl, Difluormethoxy, Chlordifluormethoxy, Chlortrifluorethoxy, Tetrafluorethoxy, Dichlordifluorethoxy, Difluortrichlorethoxy, Hexafluorpropoxy, Trifluormethylthio, Difluormethylthio, Chlordifluormethylthio, Chlortrifluorethylthio und/oder Hexafluorpropylthio substituiertes Phenoxy stehen, oder |
| $R^1$ und $R^2$ | gemeinsam für Difluormethylendioxy, Ethylendioxy, Chlortrifluorethylendioxy, Difluorethylendioxy, Methylendioxy, Trifluorethylendioxy, Difluormethylenoxydifluormethylenoxy, Tetrafluorethylendioxy, 2-Methyl-1,1,2-trifluorethylendioxy oder 2,2-Dimethylethylenoxy stehen, |
| $R^3$ und $R^4$ | gleich oder verschieden sind und für Wasserstoff, Methyl, Ethyl, n-Propyl, i-Propyl, n-Butyl, i-Butyl, sec.-Butyl, tert.-Butyl, Methoxy, Ethoxy, Trifluormethyl, Trifluormethoxy, Difluormethyl, Difluorethyl, Pentafluorethyl, Difluormethoxy, Chlordifluormethoxy, Chlortrifluorethoxy, Tetrafluorethoxy, Dichlordifluorethoxy, Difluortrichlorethoxy oder Hexafluorpropoxy stehen und |
| X | für Sauerstoff oder Schwefel steht. |

Von besonderem Interesse sind diejenigen Verbindungen der Formel (I), in welcher

| | |
|---|---|
| $R^1$ und $R^2$ | gleich oder verschieden sind und für Wasserstoff, Fluor, Chlor, Brom, Methyl, Methoxy, Methylthio, Trifluormethyl, Trifluormethoxy, Trifluormethylthio oder für gegebenenfalls durch Chlor, Methyl, Methoxy, Methylthio, Trifluormethyl, Trifluormethoxy und/oder Trifluormethylthio substituiertes Phenoxy stehen, oder |
| $R^1$ und $R^2$ | gemeinsam für Methylendioxy, Ethylendioxy, Difluormethylendioxy, Tetrafluorethylendioxy oder Chlortrifluorethylendioxy stehen, |
| $R^3$ und $R^4$ | gleich oder verschieden sind und für Wasserstoff, Methyl, Methoxy, Trifluormethyl oder Trifluormethoxy stehen und |
| X | für Sauerstoff oder Schwefel steht. |

Verwendet man nach Verfahrensvariante (a) 3-Amino-4-(4-chlorphenyl)-1,2,5-oxadiazol und 3-Trifluormethylcyclohexylisocyanat als Ausgangsstoffe, so kann der Reaktionsablauf durch das folgende Formelschema wiedergegeben werden:

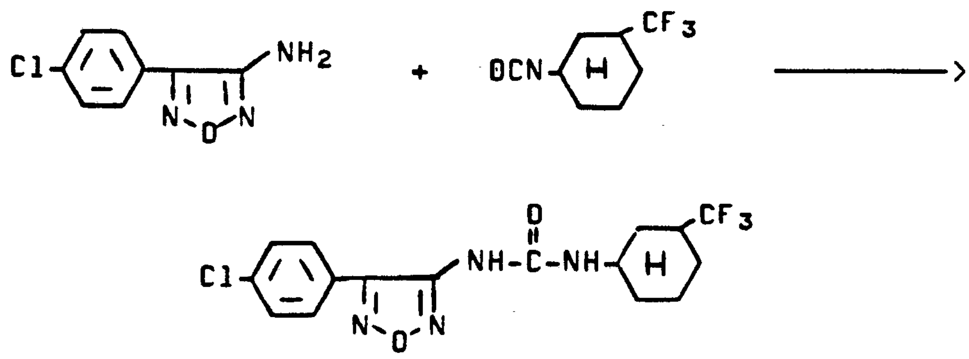

Verwendet man nach Verfahrensvariante (b) 4-(4-Chlorphenyl)-3-isocyanato-1,2,5-oxadiazol und 4-Trifluormethylcyclohexylamin als Ausgangsstoffe, so kann der Reaktionsablauf durch das folgende Formelschema wiedergegeben werden:

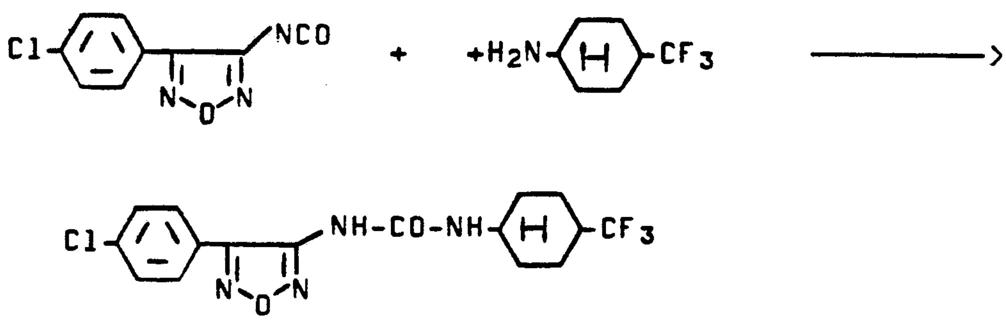

Die als Ausgangsstoffe zu verwendenden 3-Amino-1,2,5-oxadiazole der Formel (II) sind bekannt und/oder lassen sich nach literaturbekannten Verfahren und Methoden herstellen (vergl. J. Prakt. Chem. 315, 4, Seite 791 - 795 (1973)). Die Aminogruppe kann nach üblichen Verfahren in die Isocyanat- bzw. Iso-thio-cyanat-Gruppe umgewandelt werden, z. B. durch Umsetzung mit Phosgen bzw. Thiophosgen in Verdünnungsmitteln wie z. B. Toluol und/oder Pyridin, bei einer Temperatur zwischen -20° C bis +50° C (vergl. Herstellungsbeispiele und DE-A-3 326 509).

Die Verbindungen der Formeln (III) und (V) sind bekannt und/oder lassen sich nach literaturbekannten Verfahren und Methoden herstellen (vergl. z. B. DE-A-2 630 562, DE-A-2 528 162 und 'Handbuch der organischen Chemie', Band 12 und Erweiterungsbände, Beilstein).

Als Verdünnungsmittel kommen beim erfindungsgemäßen Verfehren (Varianten (a) und (b)) praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone, wie Aceton, Methyl-ethyl-, Methylisopropyl- und Methylisobutylketon, Ester, wie Essigsäuremethylester und -ethylester, Nitrile, wie z. B. Acetonitril und Propionitril, Amide, wie z. B. Dimethylacetamid und N-Methyl-pyrrolidon, sowie Tetramethylensulfon und Dimethylformamid.

Als Katalysatoren können für die Umsetzung gemäß Verfahrensvarianten (a) und (b) vorzugsweise tertiäre Amine, wie Triethylamin und 1,4-Diazabicyclo[2,2,2]-octan, sowie organische Zinn-Verbindungen, wie z. B. Dibutylzinndilaurat verwendet werden.

Die Reaktionstemperatur kann innerhalb eines größeren Bereiches variiert werden. Im allgemeinen arbeitet man bei der Verfahrensvariante (a) zwischen 20° C und 180° C, vorzugsweise zwischen 40° C und 120° C und bei der Verfahrensvariante (b) zwischen 20° C und 200° C, vorzugsweise zwischen 60° C und 190° C. Die erfindungsgemäßen Verfahrensvarianten werden im allgemeinen bei Normaldruck durchgeführt.

Zur Durchführung der erfindungsgemäßen Verfahrensvarianten werden die Ausgangsstoffe gewöhnlich in etwa äquimolaren Mengen eingesetzt. Ein Überschuß der einen oder anderen Reaktionskomponente bringt keine wesentlichen Vorteile.

Die Aufarbeitung der Reaktionsprodukte geschieht nach üblichen Methoden, z. B. durch Absaugen des ausgefallenen Produktes oder durch Herauslösung von unerwünschten Nebenprodukten aus dem Reaktionsgemisch. Zur Charakterisierung dient der Schmelzpunkt.

Die Wirkstoffe eignen sich bei guter Pflanzenverträglichkeit und günstiger Warmblütertoxizität zur Bekämpfung von tierischen Schädlingen, insbesondere Insekten und Spinnentieren, die in der Landwirtschaft,

in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z. B. Oniscus asellus, Armadilldium vulgare, Porcellio scaber.

Aus der Ordnung der Diplopoda z. B. Blaniulus guttulatus.

Aus der Ordnung der Chilopoda z. B. Geophilus carpophagus, Scutigera spec.

Aus der Ordnung der Symphyla z. B. Scutigerella immaculata.

Aus der Ordnung der Thysanura z. B. Lepisma saccharina.

Aus der Ordnung der Collembola z. B. Onychiurus armatus.

Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z. B. Forficula auricularia.

Aus der Ordnung der Isoptera z. B. Reticulitermes spp..

Aus der Ordnung der Anoplura z. B. Phylloxera vastatrix, Pemphigus spp., pediculus humanus corporis, Haematopinus spp., Linognathus spp.

Aus der Ordnung der Mallophaga z. B. Trichodectes spp., Damalinea spp.

Aus der Ordnung der Thysanoptera z. B. Hercinothrips femoralis, Thrips tabaci.

Aus der Ordnung der Heteroptera z. B. Eurygaster spp., Dysdercus intermedius, Piesma quadrata, Cimex lectularius, Rhodnius prolixus, Triatoma spp.

Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z. B. Pectinophora gossypiella, Bupalus' piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Farias insulana, Heliothis spp., Laphygma-exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp.

Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z. B. Xenopsylla cheopis, Ceratophyllus spp..

Aus der Ordnung der Arachnida z. B. Scorpio maurus, Latrodectus mactans.

Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthalino, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon,

Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyethylen-Fettsäure-Ester, Polyoxyethylen-Fettalkohol-Ether, z. B. Alkylarylpolyglykol-Ether, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische phospholipide. Weitere Additive können mineralische und vegetabile Öle sein.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen engepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die biologische Wirksamkeit der erfindungsgemäßen Verbindungen soll anhand der folgenden Beispiele erläutert werden.

**Beispiel A**

**Entwicklungshemmtest mit Tetranychus urticae (Gemeine Spinnmilbe)**

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Die Blätter der Bohnenpflanze (Phaseolus vulgaris), welche mit ca. 100 Eiern der Gemeinen Spinnmilbe belegt sind, taucht man in die entsprechend konzentrierte Wirkstoffzubereitung. Die Summe der abgetöteten Eier, Larven, Nymphen und Ruhestadien einer Generation, bezogen auf die Zahl eingesetzter Eier, ergibt die Abtötung in %. Dabei bedeutet 100 %, daß alle Tiere abgetötet wurden; 0 % bedeutet, daß keine Tiere abgetötet wurden.

Bei diesem Test zeigen z. B. bei einer Wirkstoffkonzentration von 0,0008 % die Verbindungen der Herstellungsbeispiele (3), (12) und (28) eine Abtötung von 90 bis 100 %.

**Beispiel B**

Tetranychus-Test

Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration

Mit je 50 ml der Wirkstoffzubereitung der gewünschten Konzentration werden Bohnenpflanzen (Phaseolus vulgaris) angegossen, so daß die Wirkstoffzubereitung in den Boden eindringt, ohne den Sproß zu benetzen. Der Wirkstoff wird von den Wurzeln aufgenommen und in den Sproß weitergeleitet.

Nach einem Tag läßt man Spinnmilben (Tetranychus urticae) auf die Blätter Eier ablegen und entfernt die adulten Spinnmilben. Die Abtötung der Entwicklungsstadien wird in % bestimmt. Dabei bedeutet 100 %, daß alle Spinnmilben abgetötet wurden; 0 % bedeutet, daß keine Spinnmilben abgetötet wurden.

Bei diesem Test zeigen z. B. bei einer Wirkstoffkonzentration von 0,1 % die folgenden Verbindungen der Herstellungsbeispiele (2), (3), (7), (12) und (28) eine Abtötung von 100 %.

Die Herstellung der erfindungsgemäßen Verbindungen soll durch die folgenden Herstellungsbeispiele erläutert werden:

**Beispiel 1**

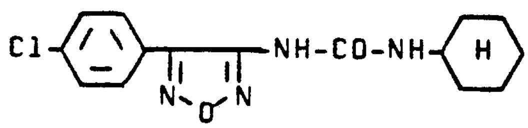

(Verfahrensvariante a)

7,84 g (0,04 Mol) 3-Amino-4-(4-chlorphenyl)-1,2,5-oxadiazol werden in 28 ml trockenem Dimethylformamid gelöst. Zu der Lösung fügt man 7 g (0,028 Mol) Cyclohexylisocyanat. Der Ansatz wird 10 Stunden bei 100°C gerührt. Beim Abkühlen auf 20°C fällt das Produkt aus. Es wird abgesaugt und mit wenig Dimethylformamid abgedeckt. Anschließend wird mit Toluol, dann mit Petrolether abgedeckt und das Produkt getrocknet.

Man erhält 9 g (70,3 % der Theorie) 1-[4-(4-Chlorphenyl)-1,2,5-oxadiazol-3-yl]-3-cyclohexylharnstoff vom Schmelzpunkt 216°C.

**Beispiel 2**

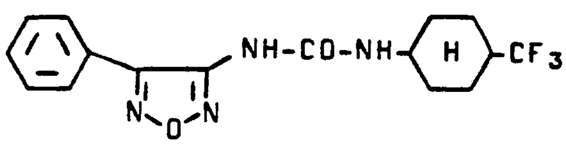

(Verfahrensvariante b)

Zu einer Lösung von 33,2 g (0,2 Mol) 4-Trifluormethylcyclohexylamin in 800 ml trockenem Toluol fügt man unter Feuchtigkeitsausschluß 37,4 g (0,2 Mol) 3-Isocyanato-4-phenyl-1,2,5- oxadiazol gelöst in 200 ml trockenem Toluol. Der Ansatz wird eine halbe Stunde bei 80°C gerührt und anschließend der größte Teil des Lösungsmittels im Vakuum abdestilliert. Der kristalline Rückstand wird abgesaugt und mit Petrolether gewaschen.

Man erhält 65,6 g (92,5 % der Theorie) 1-(4-Phenyl-1,2,5-oxadiazol-3-yl) -3-(4-trifluormethylcyclohexy])-harnstoff vom Schmelzpunkt 212°C.

Analog Beispiel 1 und 2 bzw. Verfahrensvariante (a) oder (b) werden die in der nachfolgenden Tabelle 1 aufgeführten Verbindungen der Formel (I) hergestellt:

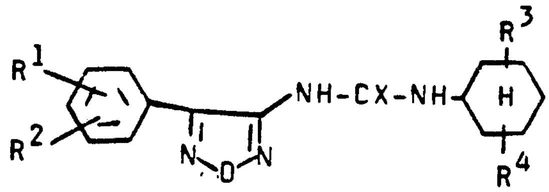

(I)

Tabelle 1

| Beisp.-Nr. | $R^1$ | $R^2$ | $R^3$ | $R^4$ | X | Schmelzpunkt [°C] |
|---|---|---|---|---|---|---|
| 3 | 4-Cl | H | 4-$CF_3$ | H | O | 203 |
| 4 | 4-Cl | H | 3-$CF_3$ | H | O | 190 |
| 5 | 4-Cl | H | 3-$CF_3$ | 5-$CF_3$ | O | 189 |
| 6 | H | H | 3-$CF_3$ | 5-$CF_3$ | O | 200 |
| 7 | H | H | 3-$CF_3$ | H | O | 171 |
| 8 | H | H | H | H | O | 196 |
| 9 | H | H | H | 4-$CH_3$ | O | 213 |
| 10 | H | H | H | 2-$CH_3$ | O | 198 |
| 11 | H | H | H | 3-$CH_3$ | O | 176 |
| 12 | 2-Cl | H | H | 4-$CF_3$ | O | 223 |
| 13 | 2-Cl | H | H | 3-$CF_3$ | O | 219 |
| 14 | 2-Cl | H | H | H | O | 212 |
| 15 | 2-Cl | H | H | 4-$CH_3$ | O | 206 |
| 16 | 4-Cl | H | H | 2-$CF_3$ | O | 208 |
| 17 | H | H | H | 2-$CF_3$ | O | 219 |
| 18 | 2-Cl | 4-Cl | H | 3-$CF_3$ | O | 193 |
| 19 | 2-Cl | 4-Cl | H | 4-$CF_3$ | O | 199 |
| 20 | 2-Cl | 4-Cl | 3-$CF_3$ | 5-$CF_3$ | O | 231 |
| 21 | 2-Cl | 4-Cl | H | 4-$CH_3$ | O | 186 |
| 22 | 2-Cl | 4-Cl | H | 2-$CH_3$ | O | 210 |
| 23 | 4-F | H | H | 4-$CF_3$ | O | 196 |
| 24 | 4-F | H | H | 3-$CF_3$ | O | 158 |
| 25 | 4-F | H | H | 4-$CH_3$ | O | 180 |
| 26 | 4-F | H | 3-$CF_3$ | 5-$CF_3$ | O | 198 |
| 27 | 4-Br | H | H | 4-$CH_3$ | O | 205 |
| 28 | 4-Br | H | H | 4-$CF_3$ | O | 200 |
| 29 | 4-F | H | H | H | O | 197 |
| 30 | 4-Br | H | H | 3-$CF_3$ | O | 207 |
| 31 | 4-Br | H | 3-$CF_3$ | 5-$CF_3$ | O | 190 |
| 32 | 4-$CF_3$ | H | H | 4-$CF_3$ | O | 156 |
| 33 | 4-$CF_3$ | H | H | 3-$CF_3$ | O | 200 |
| 34 | 4-$CF_3$ | H | 3-$CF_3$ | 5-$CF_3$ | O | 182 |
| 35 | 4-$CF_3$ | H | H | 3-$CH_3$ | O | 197 |
| 36 | 4-$CF_3O$ | H | H | 4-$CF_3$ | O | 174 |
| 37 | 4-$CF_3$-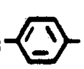-O | H | H | 4-$CF_3$ | O | 194 |
| 38 | 4-$CH_3O$ | H | H | 4-$CF_3$ | O | 212 |
| 39 | 4-$CH_3O$ | H | H | 4-$CH_3$ | O | 170 |
| 40 | 4-$CH_3O$ | H | H | 3-$CF_3$ | O | 193 - 196 |
| 41 | 4-$CH_3$ | H | 3-$CF_3$ | 5-$CF_3$ | O | 201 |
| 42 | 4-$CH_3$ | H | H | 4-$CF_3$ | O | 203 |
| 43 | 3,4-O-$CH_2$-O- | | H | 4-$CF_3$ | O | 206 |
| 44 | 4-$CH_3$ | H | H | 4-$CH_3$ | O | 197 |
| 45 | 4-$CF_3$-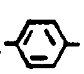-O | H | H | 3-$CF_3$ | O | 173 |
| 46 | 4-$CH_3$ | H | H | 3-$CF_3$ | O | 191 |
| 47 | 3,4-O-$CH_2$-O- | | H | H | O | 227 |
| 48 | 3,4-O-$CH_2$-O- | | H | 4-$CH_3$ | O | 205 |
| 49 | 3,4-O-$CH_2$-O- | | H | 3-$CF_3$ | O | 205 |

| | | | | | | |
|---|---|---|---|---|---|---|
| 50 | 3-Cl | 4-Cl | H | 4-$CF_3$ | O | 186 |
| 51 | 3-Cl | 4-Cl | H | 3-$CF_3$ | O | 206 |
| 52 | 3-Cl | 4-Cl | H | 4-$CH_3$ | O | 175 |
| 53 | 4-$CF_3$S | H | H | 4-$CF_3$ | O | 187 |
| 54 | 4-$CF_3$- 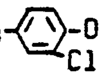 | H | H | 4-$CF_3$ | O | 187 |
| 55 | 4-$CF_3$- 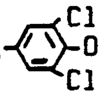 | H | H | 4-$CF_3$ | O | 182 |
| 56 | H | H | 3-CF3 | 4-$CH_3O$ | O | 186 |
| 57 | H | H | 2-$CH_3O$ | 5-$CF_3$ | O | 225 |
| 58 | 4-Cl | H | 3-$CF_3$ | 4-$CH_3O$ | O | 124 |
| 59 | 4-Cl | H | 2-$CH_3O$ | 5-$CF_3$ | O | 219 |
| 60 | 4-F | H | 3-$CF_3$ | 4-$CH_3O$ | O | 187 |
| 61 | 4-Br | H | 2-$CH_3O$ | 5-$CF_3$ | O | 220 |
| 62 | 2-Cl | 4-Cl | 3-$CF_3$ | 4-$CH_3O$ | O | 179 |
| 63 | 4-$CF_3$ | H | 3-$CF_3$ | 4-$CH_3O$ | O | 160 |
| 64 | 4-$CF_3O$ | H | 3-$CF_3$ | 4-$CH_3O$ | O | 163 |
| 65 | 4-$CF_3$- 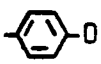 | H | 3-$CF_3$ | 4-$CH_3O$ | O | 186 |
| 66 | 4-$CF_3$- 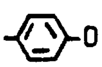 | H | 2-$CH_3O$ | 5-$CF_3$ | O | 162 |
| 67 | H | H | H | 4-$CF_3$ | S | 159 |
| 68 | H | H | 3-$CF_3$ | 5-$CF_3$ | S | 128 |
| 69 | H | H | H | 3-$CF_3$ | S | 142 |
| 70 | 3- 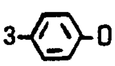 | H | H | 4-$CF_3$ | O | 209 |
| 71 | 3- 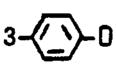 | 4-F | H | 4-$CF_3$ | O | 215 |
| 72 | 2-Cl | H | H | 4-$CF_3$ | S | 150 |
| 73 | 2-Cl | H | 3-$CF_3$ | 5-$CF_3$ | S | 150 |
| 74 | H | H | 2-$CH_3O$ | 5-$CF_3$ | S | 169 |
| 75 | H | H | 3-$CF_3$ | 4-$CH_3O$ | S | 152 |
| 76 | 3- 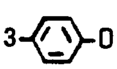 | 4-F | 3-$CF_3$ | 4-$CH_3O$ | O | 150 |
| 77 | 2-Cl | H | 2-$CH_3O$ | 5-$CF_3$ | O | 242 |
| 78 | 2-Cl | H | 2-$CH_3O$ | 5-$CF_3$ | S | 179 |
| 79 | 2-Cl | H | 3-$CF_3$ | 4-$CH_3O$ | O | 221 |
| 80 | 2-Cl | H | 3-$CF_3$ | 4-$CH_3O$ | S | 152 |
| 81 | 4-F | H | 3-$CF_3$ | 6-$CH_3O$ | O | 195 |
| 82 | H | H | 3-$CF_3$ | 4-$CH_3$ | O | 170 |
| 83 | 4-Cl | H | 3-$CF_3$ | 4-$CH_3$ | O | 165 |
| 84 | 2-Cl | H | 3-$CF_3$ | 4-$CH_3$ | O | 203 |
| 85 | 4-Br | H | 3-$CF_3$ | 4-$CH_3$ | O | 164 |
| 86 | H | H | 2-$CF_3$ | 4-$CF_3$ | O | 226 |
| 87 | 4-Cl | H | 2-$CF_3$ | 4-$CF_3$ | O | 186 |
| 88 | 2-Cl | H | 2-$CF_3$ | 4-$CF_3$ | O | 227 |
| 89 | 4-Br | H | 2-$CF_3$ | 4-$CF_3$ | O | 192 |
| 90 | H | H | 3-$CF_3$ | 4-$CH_3$ | S | 144 |
| 91 | 2-Cl | H | 3-$CF_3$ | 4-$CH_3$ | S | 139 |
| 92 | H | H | 2-$CF_3$ | 4-$CF_3$ | S | 110 |
| 93 | 2-Cl | H | 2-$CF_3$ | 4-$CF_3$ | S | 115 |
| 94 | 4-F | H | 2-$CF_3$ | 4-$CF_3$ | O | 220 |
| 95 | 4-F | H | 3-$CF_3$ | 4-$CH_3$ | O | 171 |
| 96 | 4-$F_3C$- 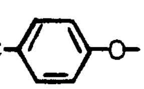 | H | 2-$CF_3$ | 4-$CF_3$ | O | 161 |

| | | | | | | |
|---|---|---|---|---|---|---|
| 97 | 2-Cl | 4-Cl | 2-$CF_3$ | 4-$CF_3$ | O | 196 |
| 98 | 4-$CF_3$ | H | 2-$CF_3$ | 4-$CF_3$ | O | 160 |
| 99 | 2-Cl | 4-Cl | 3-$CF_3$ | 4-$CH_3$ | O | 195 |
| 100 | 4-$CF_3$ | H | 3-$CF_3$ | 4-$CH_3$ | O | 142 |
| 101 | 4-$OCF_3$ | H | 2-$CF_3$ | 4-$CF_3$ | O | 176 |
| 102 | 4-$OCH_3$ | H | 2-$CF_3$ | 4-$CF_3$ | O | 152 |
| 103 | 4-$OCF_3$ | H | 3-$CF_3$ | 4-$CH_3$ | O | 130 |
| 104 | 4-$OCH_3$ | H | 3-$CF_3$ | 4-$CH_3$ | O | 184 |
| 105 | 4-$F_3C$- 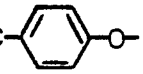 | H | 3-$CF_3$ | 4-$CH_3$ | O | 105 (Zers.) |

**Ausgangsprodukte der Formel (IV):**

**Beispiel (IV-1)**

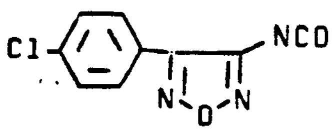

Bei 0°C bis 5°C werden 50 g Phosgen in 400 ml trockenem Toluol eingegast. In diese kalte Lösung werden bei -10°C bis +5°C eine Lösung von 58,7 g (0,3 Mol) 3-Amino-4-(4-chlorphenyl)-1,2,5-oxadiazol und 52,2 g (0,66 Mol) Pyridin in 400 ml Toluol zugetropft und 2 Stunden bei 30°C gerührt. Die ausgefallenen Salze werden unter Feuchtigkeitsausschluß abgesaugt, das Filtrat wird eingeengt und der Rückstand wird im Vakuum destilliert.

Man erhält 30,5 g (54,5 % der Theorie) 4-(4-Chlorphenyl)-3-isocyanato-1,2,5-oxadiazol vom Siedepunkt $Kp_2$.: 108°C bis 112°C.

Analog Beispiel (IV-1) können die übrigen Verbindungen der Formel (IV) erhalten werden:

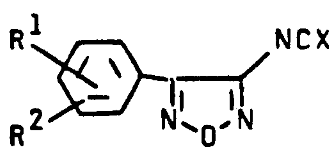

(IV)

**Tabelle 2**

| Beisp.-Nr. | $R^1$ | $R^2$ | X | Physikal. Konstante |
|---|---|---|---|---|
| IV-2 | H | H | O | Fp: 53 - 54°C |
| IV-3 | H | 2-Cl | O | $n_D^{20}$: 1,5646 |
| IV-4 | 2-Cl | H | S | $n_D^{20}$: 1,6242 |
| IV-5 | 4-F | H | O | Fp: 80°C |
| IV-6 | 4-Br | H | O | Fp: 82°C |
| IV-7 | 4-$CF_3$ | H | O | $n_D^{20}$: 1,5079 |
| IV-8 | 4-$CF_3O$ | H | O | $n_D^{20}$: 1,4986 |
| IV-9 | 4-$CH_3O$ | H | O | Fp: 42°C |
| IV-10 | 4-$CH_3$ | H | O | Fp: 52°C |
| IV-11 | 4-$CF_3S$ | H | O | $n_D^{20}$: 1,5378 |
| IV-12 | 2-Cl | 4-Cl | O | Fp: 67°C |
| IV-13 | 3-Cl | 4-Cl | O | Fp: 40°C |
| IV-14 | 4-$CF_3$- 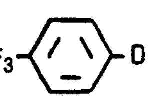 | H | O | $n_D^{20}$: 1,5585 |
| IV-15 | 4-$CF_3$- 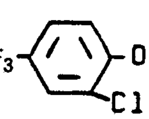 | H | O | $n_D^{20}$: 1,5663 |
| IV-16 | 4-$CF_3$- 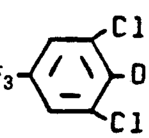 | H | O | Fp: 70°C |

| | | | | |
|---|---|---|---|---|
| IV-17 | 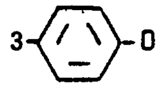 | H | O | $n_D^{20}$: 1,5988 |
| IV-18 | 4-F | 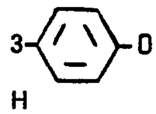 | | $n_D^{20}$: 1,5860 |
| IV-19 | H | H | S | $n_D^{20}$: 1,6298 |

**Patentansprüche**

1. Substituierte Furazane der Formel (I)

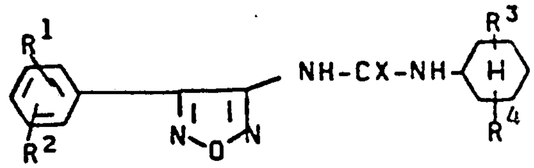

(I)

in welcher

| | |
|---|---|
| $R^1$ à $R^2$ | gleich oder verschieden sind und für Wasserstoff, Halogen, $C_1$-$C_6$-Alkyl, $C_1$-$C_6$-Alkoxy, Cl-$C_6$-Alkylthio, Halogen-$C_1$-$C_6$-Alkyl, Halogen-$C_1$-$C_6$-Alkoxy, Halogen-$C_1$-$C_6$-Alkylthio oder für gegebenenfalls durch Halogen, Nitro, Cyano, $C_1$-$C_4$-Alkyl, $C_1$-$C_4$-Alkoxy, $C_1$-$C_4$-Alkylthio, Halogen-$C_1$-$C_4$-Alkyl, Halogen-$C_1$-$C_4$-Alkoxy und/oder Halogen-$C_1$-$C_4$-Alkylthio substituiertes Aryloxy mit 6 bis 10 Kohlenstoffatomen im Arylteil stehen, oder |
| $R^1$ und $R^2$ | gemeinsam für einen gegebenenfalls durch Halogen und/oder $C_1$-$C_2$-Alkyl substituierten $C_1$-$C_3$-Alkylen-Rest, welcher durch 1 oder 2 Sauerstoffatome unterbrochen wird, stehen, |
| $R^3$ und $R^4$ | gleich oder verschieden sind und für Wasserstoff, $C_1$-$C_6$-Alkyl, $C_1$-$C_6$-Alkoxy, Halogen-$C_1$-$C_6$-Alkyl oder Halogen-$C_1$-$C_4$-Alkoxy stehen und |
| X | für Sauerstoff oder Schwefel steht. |

2. Verbindungen der Formel (I) gemäß Anspruch 1, in welcher

| | |
|---|---|
| $R^1$ und $R^2$ | gleich oder verschieden sind und für Wasserstoff, Fluor, Chlor, Brom $C_1$-$C_4$-Alkyl, $C_1$-$C_4$-Alkoxy, $C_1$-$C_4$-Alkylthio, Halogen-$C_1$-$C_4$-Alkyl, Halogen-$C_1$-$C_4$-Alkoxy, Halogen-$C_1$-$C_4$-Alkylthio oder für gegebenenfalls durch Halogen, Nitro, Cyano, $C_1$-$C_4$-Alkyl, $C_1$-$C_4$-Alkoxy, $C_1$-$C_4$-Alkylthio, Halogen-$C_1$-$C_4$-Alkyl, Halogen-$C_1$-$C_4$-Alkoxy und/oder Halogen-$C_1$-$C_4$-Alkylthio substituiertes Phenoxy stehen, oder |
| $R^1$ und $R^2$ | gemeinsam für einen gegebenenfalls durch Fluor, Chlor und/oder Methyl substituierten $C_1$-$C_3$-Alkylen-Rest, welcher durch 1 oder 2 Sauerstoffatome unterbrochen wird, stehen, |
| $R^3$ und $R^4$ | gleich oder verschieden sind und für Wasserstoff, $C_1$-$C_4$-Alkyl, $C_1$-$C_4$-Alkoxy, Halogen-$C_1$-$C_4$-Alkyl oder Halogen-$C_1$-$C_4$-Alkoxy stehen und |
| X | für Sauerstoff oder Schwefel steht. |

3. Verbindungen der Formel (I) gemäß Anspruch 1, in welcher

| | |
|---|---|
| $R^1$ und $R^2$ | gleich oder verschieden sind und für Wasserstoff, Fluor, Chlor, Brom, Methyl, Methoxy, Methylthio, Trifluormethyl, Trifluormethoxy, Trifluormethylthio oder für gegebenenfalls durch Chlor, Methyl, Methoxy, Methylthio, Trifluormethyl, Trifluormethoxy und/oder Trifluormethylthio substituiertes Phenoxy stehen, oder |
| $R^1$ und $R^2$ | gemeinsam für Methylendioxy, Ethylendioxy, Difluormethylendioxy, Tetrafluorethylendioxy oder Chlortrifluorethylendioxy stehen, |
| $R^3$ und $R^4$ | gleich oder verschieden sind und für Wasserstoff, Methyl, Methoxy, Trifluormethyl oder Trifluormethoxy stehen und |
| X | für Sauerstoff oder Schwefel steht. |

4. Verbindungen der Formel (I) gemäß Anspruch 1

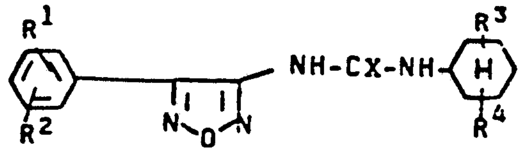 (I)

in welcher

(a) $R^1$ für Wasserstoff, $R^2$ für 4-Chlor, $R^3$ für Wasserstoff, $R^4$ für 4-$CF_3$ und X für Sauerstoff stehen, oder
(b) $R^1$ und $R^2$ für Wasserstoff, $R^3$ für Wasserstoff, $R^4$ für 4-$CF_3$ und X für Sauerstoff stehen, oder
(c) $R^1$ und $R^2$ für Wasserstoff, $R^3$ für Wasserstoff, $R^4$ für 3-$CF_3$ und X für Sauerstoff stehen, oder
(d) $R^1$ für Wasserstoff, $R^2$ für 2-Chlor, $R^3$ für Wasserstoff, $R^4$ für 3-$CF_3$ und X für Sauerstoff stehen, oder
(e) $R^1$ für Wasserstoff, $R^2$ für 4-Brom, $R^3$ für Wasserstoff, $R^4$ für 4-$CF_3$ und X für Sauerstoff stehen.

5. Verfahren zur Herstellung der substituierten Furazane der Formel (I)

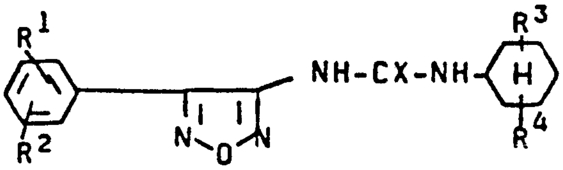 (I)

in welcher

$R^1$ und $R^2$ gleich oder verschieden sind und für Wasserstoff, Halogen, $C_1$-$C_6$-Alkyl, $C_1$-$C_6$-Alkoxy, $C_1$-$C_6$-Alkylthio, Halogen-$C_1$-$C_6$-Alkyl, Halogen-$C_1$-$C_6$-Alkoxy, Halogen-$C_1$-$C_6$-Alkylthio oder für gegebenenfalls durch Halogen, Nitro, Cyano, $C_1$-$C_4$-Alkyl, $C_1$-$C_4$-Alkoxy, $C_1$-$C_4$-Alkylthio, Halogen-$C_1$-$C_4$-Alkyl, Halogen-$C_1$-$C_4$-Alkoxy und/oder Halogen-$C_1$-$C_4$-Alkylthio substituiertes Aryloxy mit 6 bis 10 Kohlenstoffatomen im Arylteil stehen, oder

$R^1$ und $R^2$ gemeinsam für einen gegebenenfalls durch Halogen und/oder $C_1$-$C_2$-Alkyl substituierten $C_1$-$C_3$-Alkylen-Rest, welcher durch 1 oder 2 Sauerstoffatome unterbrochen wird, stehen,

$R^3$ und $R^4$ gleich oder verschieden sind und für Wasserstoff, $C_1$-$C_6$-Alkyl, $C_1$-$C_6$-Alkoxy, Halogen-$C_1$-$C_6$-Alkyl oder Halogen-$C_1$-$C_4$-Alkoxy stehen und

X für Sauerstoff oder Schwefel steht,

dadurch gekennzeichnet, daß man

a) 3-Amino-1,2,5-oxadiazole der Formel (II)

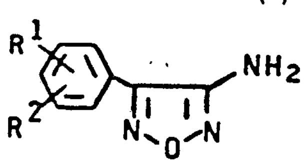 (II)

in welcher
$R^1$ und $R^2$ die oben angegebenen Bedeutungen haben,
mit Iso(thio)cyanaten der Formel (III)

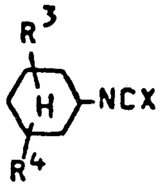 (III)

in welcher
X, $R^3$ und $R^4$ die oben angegebenen Bedeutungen haben,
gegebenenfalls in Gegenwart von Katalysatoren und gegebenenfalls in Gegewart von Verdünnungsmitteln umsetzt, oder

b) 3-Iso(thio)cyanato-1,2,5-oxadiazole der Formel (IV),

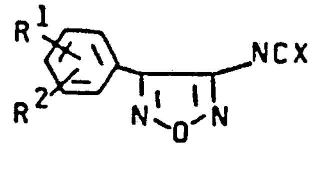

(IV)

in welcher

X, $R^1$ und $R^2$ die oben angegebenen Bedeutungen haben,
mit Aminen der Formel (V)

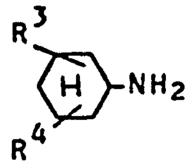

(V)

in welcher

$R^3$ und $R^4$ die oben angegebenen Bedeutungen haben,
gegebenenfalls in Gegenwart von Katalysatoren und gegebenenfalls in Gegenwart von Verdünnungsmitteln umsetzt.

6. Schädlingsbekämpfungsmittel, gekennzeichnet durch einen Gehalt an mindestens einer Verbindung der Formel (I) gemäß Anspruch 1 oder 5.

7. Verwendung von Verbindungen der Formel (I) gemäß Anspruch 1 oder 5 zur Bekämpfung von Schädlingen, insbesondere Insekten und Akariden.

8. Verfahren zur Bekämpfung von Schädlingen, dadurch gekennzeichnet, daß man Verbindungen der Formel (I) gemäß Anspruch 1 oder 5 auf die Schädlinge, vorzugsweise Insekten oder Akariden oder ihren Lebensraum einwirken läßt.

9. Verfahren zur Herstellung von Schädlingsbekämpfungsmitteln, dadurch gekennzeichnet, daß man Verbindungen der Formel (I) gemäß Anspruch 1 oder 5 mit Streckmitteln und/oder oberflächenaktiven Mitteln vermischt.

## Claims

1. Substituted furazanes of the formula (I)

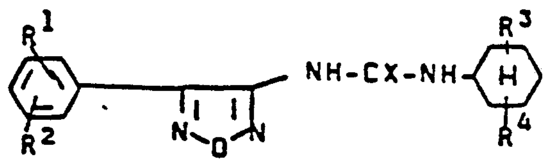

(I)

in which

$R^1$ and $R^2$ are identical or different and represent hydrogen, halogen, $C_1$-$C_6$-alkyl, $C_1$-$C_6$-alkoxy, $C_1$-$C_6$-alkylthio, halogeno-$C_1$-$C_6$-alkyl, halogeno-$C_1$-$C_6$-alkoxy or halogeno-$C_1$-$C_6$-alkylthio, or represent aryloxy which has 6 to 10 carbon atoms in the aryl part and is optionally substituted by halogen, nitro, cyano, $C_1$-$C_4$-alkyl, $C_1$-$C_4$-alkoxy, $C_1$-$C_4$-alkylthio, halogeno-$C_1$-$C_4$-alkyl, halogeno-$C_1$-$C_4$-alkoxy and/or halogeno-$C_1$-$C_4$-alkylthio, or

$R^1$ and $R^2$ together represent a $C_1$-$C_3$-alkylene radical which is interrupted by 1 or 2 oxygen atoms and is optionally substituted by halogen and/or $C_1$-$C_2$-alkyl,

$R^3$ and $R^4$ are identical or different and represent hydrogen, $C_1$-$C_6$-alkyl, $C_1$-$C_6$-alkoxy, halogeno-$C_1$-$C_6$-alkyl or halogeno-$C_1$-$C_4$-alkoxy and

X represents oxygen or sulphur.

2. Compounds of the formula (I) according to Claim 1, in which

$R^1$ and $R^2$ are identical or different and represent hydrogen, fluorine, chlorine, bromine, $C_1$-$C_4$alkyl, $C_1$-$C_4$-alkoxy, $C_1$-$C_4$-alkylthio, halogeno-$C_1$-$C_4$-alkyl, halogeno-$C_1$-$C_4$-alkoxy or halogeno-$C_1$-$C_4$-alkylthio, or represent phenoxy which is optionally substituted by halogen, nitro, cyano, $C_1$-$C_4$-alkyl, $C_1$-$C_4$-alkoxy, $C_1$-$C_4$-alkylthio, halogeno-$C_1$-$C_4$-alkyl, halogeno-$C_1$-$C_4$-alkoxy and/or halogeno-$C_1$-$C_4$-

alkylthio, or

$R^1$ and $R^2$ together represent a $C_1$-$C_3$-alkylene radical which is interrupted by 1 or 2 oxygen atoms and is optionally substituted by fluorine, chlorine and/or methyl,

$R^3$ and $R^4$ are identical or different and represent hydrogen, $C_1$-$C_4$-alkyl, $C_1$-$C_4$-alkoxy, halogeno-$C_1$-$C_4$-alkyl or halogeno-$C_1$-$C_4$-alkoxy and

X represents oxygen or sulphur.

3. Compounds of the formula (I) according to Claim 1, in which

$R^1$ and $R^2$ are identical or different and represent hydrogen, fluorine, chlorine, bromine, methyl, methoxy, methylthio, trifluoromethyl, trifluoromethoxy or trifluoromethylthio, or represent phenoxy which is optionally substituted by chlorine, methyl, methoxy, methylthio, trifluoromethyl, trifluoromethoxy and/or trifluoromethylthio, or

$R^1$ and $R^2$ together represent methylenedioxy, ethylenedioxy, difluoromethylenedioxy, tetrafluoroethylenedioxy or chlorotrifluoroethylenedioxy,

$R^3$ and $R^4$ are identical or different and represent hydrogen, methyl, methoxy, trifluoromethyl or trifluoromethoxy and

X represents oxygen or sulphur.

4. Compounds of the formula (I) according to Claim 1

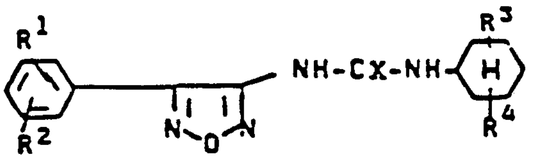

(I)

in which

(a) $R^1$ represents hydrogen, $R^2$ represents 4-chlorine, $R^3$ represents hydrogen, $R^4$ represents 4-$CF_3$ and X represents oxygen, or

(b) $R^1$ and $R^2$ represent hydrogen, $R^3$ represents hydrogen, $R^4$ represents 4-$CF_3$ and X represents oxygen, or

(c) $R^1$ and $R^2$ represent hydrogen, $R^3$ represents hydrogen, $R^4$ represents 3-$CF_3$ and X represents oxygen, or

(d) $R^1$ represents hydrogen, $R^2$ represents 2-chlorine, $R^3$ represents hydrogen, $R^4$ represents 3-$CF_3$ and X represents oxygen, or

(e) $R^1$ represents hydrogen, $R^2$ represents 4-bromine, $R^3$ represents hydrogen, $R^4$ represents 4-$CF_3$ and X represents oxygen.

5. Process for the preparation of the substituted furazanes of the formula (I)

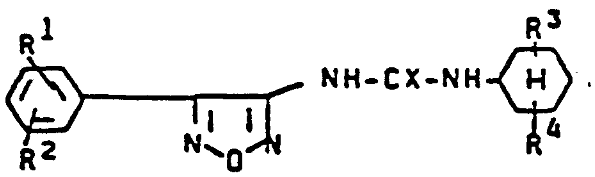

(I)

in which

$R^1$ and $R^2$ are identical or different and represent hydrogen, halogen, $C_1$-$C_6$-alkyl, $C_1$-$C_6$-alkoxy, $C_1$-$C_6$-alkylthio, halogeno-$C_1$-$C_6$-alkyl, halogeno-$C_1$-$C_6$-alkoxy, halogeno-$C_1$-$C_6$-alkylthio or aryloxy which has 6 to 10 carbon atoms in the aryl part and is optionally substituted by halogen, nitro, cyano, $C_1$-$C_4$-alkyl, $C_1$-$C_4$-alkoxy, $C_1$-$C_4$-alkylthio, halogeno-$C_1$-$C_4$-alkyl, halogeno-$C_1$-$C_4$-alkoxy and/or halogeno-$C_1$-$C_4$-alkylthio or

$R^1$ and $R^2$ together represent a $C_1$-$C_3$-alkylene radical which is interrupted by 1 or 2 oxygen atoms and is optionally substituted by halogen and/or $C_1$-$C_2$-alkyl,

$R^3$ and $R^4$ are identical or different and represent hydrogen, $C_1$-$C_6$-alkyl, $C_1$-$C_6$-alkoxy, halogeno-$C_1$-$C_6$-alkyl or halogeno-$C_1$-$C_4$-alkoxy and

X represents oxygen or sulphur,

characterised in that

a) 3-amino-1,2,5-oxadiazoles of the formula (II)

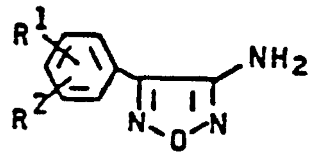

(II)

in which

$R^1$ and $R^2$ have the abovementioned meanings, are reacted with iso(thio)cyanates of the formula (III)

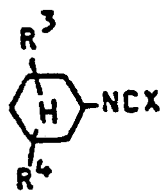

(III)

in which

X, $R^3$ and $R^4$ have the abovementioned meanings, if appropriate in the presence of catalysts and if appropriate in the presence of diluents, or

b) 3-iso(thio)cyanato-1,2,5-oxadiazoles of the formula (IV)

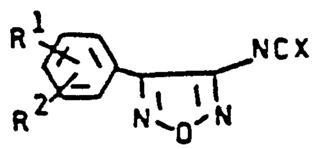

(IV)

in which

X, $R^1$ and $R^2$ have the abovementioned meanings, are reacted with amines of the formula (V)

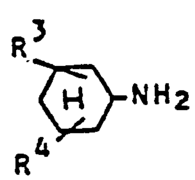

(V)

in which

$R^3$ and $R^4$ have the abovementioned meanings, if appropriate in the presence of catalysts and if appropriate in the presence of diluents.

6. Agents for combating pests, characterised in that they contain at least one compound of the formula (I) according to Claim 1 or 5.

7. Use of compounds of the formula (I) according to Claim 1 or 5 for combating pests, in particular insects and acarides.

8. Method of combating pests, characterised in that compounds of the formula (I) according to Claim 1 or 5 are allowed to act on the pests, preferably insects or acarides, or their environment.

9. Process for the preparation of agents for combating pests, characterised in that compounds of the formula (I) according to Claim 1 or 5 are mixed with extenders and/or surface-active agents.

**Revendications**

1. Furazannes substitués de formule (I)

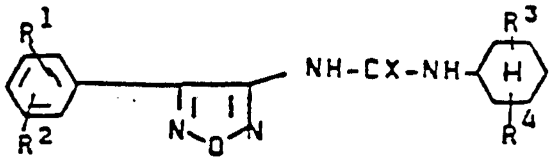

(I)

dans laquelle

$R^1$ et $R^2$ sont identiques ou différents et désignent un atome d'hydrogène, un atome d'halogène, un groupe alkyle en $C_1$ - $C_6$, un groupe alcoxy en $C_1$ - $C_6$, un groupe alkylthio en $C_1$ - $C_6$, un groupe halogénoalkyle en $C_1$ - $C_6$, un groupe halogénoalcoxy en $C_1$ - $C_6$, un groupe halogénoalkylthio en $C_1$ - $C_6$ ou un groupe aryloxy comportant 6 à 10 atomes de carbone dans la partie aryle et portant, le cas échéant, un ou plusieurs substituants halogéno, nitro, cyano, alkyle en $C_1$ - $C_4$, alcoxy en $C_1$ - $C_4$, alkylthio en $C_1$ - $C_4$, halogénoalkyle en $C_1$ - $C_4$, halogénoalcoxy en $C_1$ - $C_4$ et/ou halogénoalkylthio en $C_1$ - $C_4$ ou bien

$R^1$ et $R^2$ représentent ensemble un reste alkylène en $C_1$ - $C_3$ interrompu par 1 ou 2 atomes d'oxygène et portant le cas échéant un ou plusieurs substituants halogéno ou alkyle en $C_1$ - $C_2$,

$R^3$ et $R^4$ sont identiques ou différents et représentent un atome d'hydrogène, un groupe alkyle en $C_1$ - $C_6$, un groupe alcoxy en $C_1$ - $C_6$, un groupe halogénoalkyle en $C_1$ - $C_6$ ou halogénoalcoxy en $C_1$ - $C_4$ et

X représente un atome d'oxygène ou de soufre.

2. Composés de formule (I) selon la revendication 1, dans laquelle

$R^1$ et $R^2$ sont identiques ou différents et représentent l'hydrogène, le fluor, le chlore, le brome, un groupe alkyle en $C_1$ - $C_4$, alcoxy en $C_1$ - $C_4$, alkylthio en $C_1$ - $C_4$, halogénoalkyle en $C_1$ - $C_4$, halogénoalcoxy en $C_1$ - $C_4$, halogénoalkylthio en $C_1$ - $C_4$ ou un groupe phénoxy portant, le cas échéant, au moins un substituant halogéno, nitro, cyano, alkyle en $C_1$ - $C_4$, alcoxy en $C_1$ - $C_4$, alkylthio en $C_1$ - $C_4$, halogénoalkyle en $C_1$ - $C_4$, halogénoalcoxy en $C_1$ - $C_4$ et/ou halogénoalkylthio en $C_1$ - $C_4$, ou bien

$R^1$ et $R^2$ représentent ensemble un reste alkylène éventuellement fluoré, chloré et/ou méthylé, ledit reste alkylène étant interrompu par 1 ou 2 atomes d'oxygène,

$R^3$ et $R^4$ sont identiques ou différents et représentent l'hydrogène, un groupe alkyle en $C_1$ - $C_4$, alcoxy en $C_1$ - $C_4$, halogénoalkyle en $C_1$ - $C_4$ ou halogénoalcoxy en $C_1$ - $C_4$ et

X représente l'oxygène ou le soufre.

3. Composés de formule (I) selon la revendication 1, dans laquelle

$R^1$ et $R^2$ sont identiques ou différents et représentent l'hydrogène, le fluor, le chlore, le brome, un groupe méthyle, méthoxy, méthylthio, trifluorométhyle, trifluorométhoxy, trifluorométhylthio ou phénoxy portant éventuellement au moins un substituant chloro, méthyle, méthoxy, méthylthio, trifluorométhyle, trifluorométhoxy et/ou trifluorométhylthio, ou bien,

$R^1$ et $R^2$ représentent ensemble un groupement méthylènedioxy, éthylènedioxy, difluorométhylènedioxy, tétrafluoréthylènedioxy ou chlorotrifluoréthylènedioxy,

$R^3$ et $R^4$ sont identiques ou différents et représentent l'hydrogène, un groupe méthyle, méthoxy, trifluorométhyle ou trifluorométhoxy et

X représente l'oxygène ou le soufre.

4. Composés de formule (I) selon la revendication 1

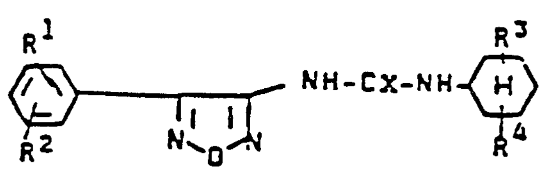

(I)

dans laquelle

(a) $R^1$ représente l'hydrogène, $R^2$ 4-chloro, $R^3$ l'hydrogène, $R^4$ 4-$CF_3$ et X l'oxygène ou bien

(b) $R^1$ et $R^2$ représentent l'hydrogène, $R^3$ l'hydrogène, $R^4$ 4-$CF_3$ et X l'oxygène ou bien
(c) $R^1$ et $R^2$ représentent l'hydrogène, $R^3$ l'hydrogène, $R^4$ 3-$CF_3$ et X l'oxygène ou bien
(d) $R^1$ représente l'hydrogène, $R^2$ 2-chloro, $R^3$ l'hydrogène, $R^4$ 3-$CF_3$ et X l'oxygène ou bien
(e) $R^1$ représente l'hydrogène, $R^2$ 4-bromo, $R^3$ l'hydrogène, $R^4$ 4-$CF_3$ et X l'oxygène.

5. Procédé de préparation de furazannes substitués de formule (I)

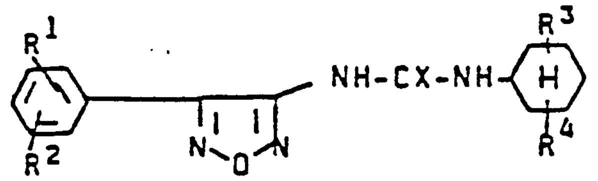

(I)

dans laquelle

$R^1$ et $R^2$ sont identiques ou différents et désignent un atome d'hydrogène, un atome d'halogène, un groupe alkyle en $C_1$ - $C_6$, un groupe alcoxy en $C_1$ - $C_6$, un groupe alkylthio en $C_1$ - $C_6$, un groupe halogénoalkyle en $C_1$ - $C_6$, un groupe halogénoalcoxy en $C_1$ - $C_6$ un groupe halogénoalkylthio en $C_1$ - $C_6$ ou un groupe aryloxy comportant 6 à 10 atomes de carbone dans la partie aryle et portant, le cas échéant, un ou plusieurs substituants halogéno, nitro, cyano, alkyle en $C_1$ - $C_4$, alcoxy en $C_1$ - $C_4$, alkylthio en $C_1$ - $C_4$, halogénoalkyle en $C_1$ - $C_4$, halogénoalcoxy en $C_1$ - $C_4$ et/ou halogénoalkylthio en $C_1$ - $C_4$ ou bien

$R^1$ et $R^2$ représentent ensemble un reste alkylène en $C_1$ - $C_3$ interrompu par 1 ou 2 atomes d'oxygène et portant, le cas échéant, un ou plusieurs substituants halogéno ou alkyle en $C_1$ - $C_2$

$R^3$ et $R^4$ sont identiques ou différents et représentent un atome d'hydrogène, un groupe alkyle en $C_1$ - $C_6$, un groupe alcoxy en $C_1$ - $C_6$, un groupe halogénoalkyle en $C_1$ - $C_6$ ou halogénoalcoxy en $C_1$ - $C_4$ et

X représente un atome d'oxygène ou de soufre, caractérisé en ce qu'on fait réagir

a) des 3-amino-1,2,5-oxadiazoles de formule (II)

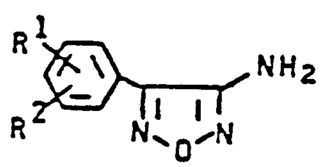

(II)

dans laquelle

$R^1$ et $R^2$ ont les significations indiquées précédemment avec des iso(thio)cyanates de formule (III)

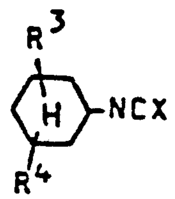

(III)

dans laquelle

X, $R^3$ et $R^4$ ont les significations indiquées précédemment, le cas échéant en présence de catalyseurs et le cas échéant en présence de diluants, ou

b) des 3-iso(thio)cyanato -1,2,5-oxadiazoles de formule (IV)

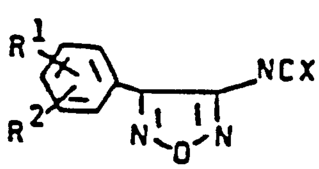

(IV)

dans laquelle

X, $R^1$ et $R^2$ ont les significations indiquées précédemment avec des amines de formule (V)

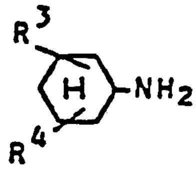

(V)

dans laquelle

$R^3$ et $R^4$ ont les significations indiquées précédemment, le cas échéant en présence de catalyseurs et le cas échéant en présence de diluants.

6. Agents de lutte contre les parasites ou animaux nuisibles caractérisés par une teneur en au moins un composé de formule (I) selon la revendication 1 ou 5.

7. Utilisation des composés de formule (I) selon la revendication 1 ou 5 pour la lutte contre les parasites ou animaux nuisibles en particulier les insectes et acariens.

8. Procédé de lutte contre les parasites ou animaux nuisibles, caractérisé en ce qu'on laisse agir des composés de formule I selon la revendication 1 ou 5 sur les parasites ou animaux nuisibles, de préférence sur les insectes ou acariens ou sur leur espace vital.

9. Procédé de préparation d'agents de lutte contre les parasites ou animaux nuisibles, caractérisé en ce que l'on mélange des composés de formule I selon la revendication 1 ou 5 avec des diluants et/ou des agents tensio-actifs.